# EUROPÄISCHE PATENTSCHRIFT

(11) **EP 2 537 835 B1**
(45) Veröffentlichungstag und Bekanntmachung des Hinweises auf die Patenterteilung: **30.04.2014**
(21) Anmeldenummer: 12183433.7
(22) Anmeldetag: 28.02.2008
(51) Int. Cl.: C07D 295/13

(54) **Neues Verfahren zur Herstellung von TETA über EDDN**
New method for producing TETA by means of EDDN
Nouveau procédé destiné à la fabrication de TETA par EDDN

(30) Priorität: 01.03.2007 EP 07103295
(43) Veröffentlichungstag der Anmeldung: 26.12.2012
(62) Teilanmeldung aus: 08717210.2
(73) Patentinhaber: BASF SE, 67056 Ludwigshafen (DE)
(72) Erfinder: Dahmen, Kirsten, 67251 Freinsheim (DE); Oftring, Alfred, 67098 Bad Dürkheim (DE); Baumann, Katrin, 68529 Mannheim (DE); Hugo, Randolf, 67246 Dirmstein (DE); Hahn, Thilo, 67292 Kirchheimbolanden (DE); Melder, Johann-Peter, 67459 Böhl-Iggelheim (DE)

(56) Entgegenhaltungen:
- EP-A1- 1 209 146
- US-A1- 2006 041 107

## Beschreibung

Die Erfindung betrifft ein Verfahren zur Herstellung von Triethylentetraamin (TETA) durch Hydrierung von Ethylendiamindiacetonitril (EDDN) an einem Katalysator, wobei EDDN durch Umsetzung von Ethylendiamin (EDA) mit Formaldehyd und Blausäure (HCN) hergestellt wird. Gegebenenfalls kann EDDN auch als Bestandteil eines Aminonitrilgemisches vorliegen, das zusätzlich Ethylendiaminmonoacetonitril (EDMN) enthält. Durch Rückführung von bei der Hydrierung gegebenenfalls erhaltenem Diethylentriamin (DETA) können im Aminonitrilgemisch zusätzlich Diethylentriaminmonoacetonitril (DETMN) oder Diethylentriamindiacetonitril (DETDN) enthalten sein. Durch Hydrierung dieser weiteren Aminonitrile wird zusätzlich Tetraethylenpentaamin (TEPA) erhalten.

Es ist generell bekannt, dass aliphatische Nitrile, die gegebenenfalls noch mit weiteren funktionellen Gruppen substituiert sind, in Gegenwart von Katalysatoren zu den entsprechenden Aminen hydriert werden können. Wie nachfolgend aufgezeigt, sind solche Hydrierverfahren auch für diverse Aminonitrile zur Herstellung von einigen Aminen bekannt. Bis jetzt ist jedoch nirgendwo beschrieben worden, dass auch TETA ausgehend von dem Aminonitril EDDN oder gegebenenfalls von einem Aminonitrilgemisch enthaltend EDDN und EDMN durch direkte Hydrierung des Aminonitrils hergestellt werden kann. Die bisher bekannten Verfahren zur Herstellung von TETA sind jedoch, wie nachfolgend aufgeführt, mit Nachteilen verbunden.

Im Stand der Technik sind zahlreiche Verfahren zur Hydrierung der α-Aminonitrile Aminoacetonitril (AAN) und Iminodiacetonitril (IDAN) oder von β-Aminonitrilen beschrieben. So ist es bekannt, dass die Hydrierung von β-Aminonitrilen in aller Regel problemlos verläuft, während die Hydrierung von α-Aminonitrilen mit dem Auftreten von zahlreichen Nachteilen verbunden ist, wie der Hydrogenolyse der C-CN-Bindung oder der R₂N-C-Bindung. ""Handbook of Heterogeneous Catalytic Hydrogenation for Organic Synthesis, S. 213 bis 215" zeigt die Problematik der Hydrierung von α-Aminonitrilen anhand von α-Alkylaminonitrilen oder cyclischen α-Aminonitrilen im Vergleich zu β-Aminonitrilen auf. Die bekannten Stabilitätsprobleme der α-Aminonitrile sind vermutlich der Hauptgrund dafür, warum bis heute nur die Hydrierung der α-Aminonitrile AAN oder IDAN zu EDA (Ethylendiamin) bzw. DETA (Diethylentriamin) genauer beschrieben wird. Großtechnisch werden EDA oder DETA jedoch durch die nachfolgend beschriebenen EDC- oder MEA-Verfahren hergestellt. Für höhere α-Aminonitrile ist eine entsprechende Hydrierung jedoch nicht bekannt.

In DE-A 3 003 729 wird ein Verfahren zum Hydrieren von aliphatischen Nitrilen, Alkylenoxynitrilen und Alkylenaminonitrilen zu primären Aminen in Anwesenheit eines Lösungsmittelsystems an einem Kobalt- oder Rutheniumkatalysator beschrieben. Das verwendete Lösungsmittelsystem enthält neben Wasser und Ammoniak einen Ether oder Polyether. Die als Edukte einsetzbare Alkylenaminonitrile oder Alkylenoxynitrile sind jeweils über komplexe allgemeine Formeln definiert. Unter anderem sind als konkrete Verbindungen oder Beispiele, die zum entsprechenden Diamin hydriert werden können, Ethylendiamindipropionitril (EDDPN; auch als N,N'-Bis(cyanoethyl)-ethylendiamin bezeichnet) oder 3,3'-(Ethylendioxy)-dipropionitril aufgeführt. DE-A 3 003 729 offenbart hingegen keinen Hinweis auf die Verwendung von Einzelverbindungen von EDA-Derivaten mit Cyanomethylsubstituenten, wie EDDN oder EDMN. Letztgenanntes fällt zudem nicht unter die allgemeine Definition von Alkylenaminonitrilen gemäß diesem Dokument.

EP-A 0 382 508 beschreibt ein Verfahren zur chargenweisen Herstellung von nicht-cyclischen, aliphatischen Polyaminen durch Hydrierung von nicht-cyclischen, aliphatischen Polynitrilen in flüssiger Phase an Raney-Kobalt-Katalysatoren, vorzugsweise in Gegenwart von wasserfreiem Ammoniak. Hierbei wird eine Polynitrillösung einer Reaktionszone zugeführt, die den Raney-Kobalt-Katalysator in einer im Wesentlichen sauerstofffreien Atmosphäre enthält. Während des gesamten Reaktionszeitraums wird die Polynitrillösung mit einer Rate zugeführt, die nicht größer ist als die maximale Rate, mit der das Polynitril mit dem Wasserstoff in der Reaktionszone reagiert. Mit diesem Verfahren können Polyamine aus Polynitrilen, wie Iminodiacetonitril (IDAN), Nitrilotriacetonitril (NTAN), Ethylendiamintetraacetonitril (EDTN) oder weitere nicht näher spezifizierte Verbindungen mit 2 oder mehr Cyanogruppen hergestellt werden. Das direkte Hydrierungsprodukt von IDAN ist Diethylentriamin (DETA).

EP-A 212 986 betrifft ein weiteres Verfahren, bei dem die gleichen aliphatischen Polynitrile wie in EP-A 0 382 508 in Gegenwart eines im Eduktstrom enthaltenen, flüssigen primären oder sekundären Amins an einem granularen Raney-Kobalt-Katalysator zu den entsprechenden Polyaminen hydriert werden können. Unter anderem ist als zwingend vorhandene Aminokomponente Ethylendiamin (EDA) neben zahlreichen weiteren primären oder sekundären Aminen aufgeführt.

EP-A 1 209 146 betrifft ein weiteres Verfahren zur kontinuierlichen Hydrierung von Nitrilen zu primären Aminen, wobei die jeweiligen Nitrile in flüssiger Phase an einem suspendierten, aktivierten Raney-Katalysator auf Basis einer Legierung aus Aluminium eingesetzt werden und die Reaktion in Abwesenheit von Ammoniak und basischen Alkali- oder Erdalkaliverbindungen durchgeführt wird. Als Nitrile können neben vielen anderen auch IDAN, EDTN, EDDPN oder Ethylendiaminmonopropionitril (EDMPN) zu den entsprechenden Ethylenaminen umgesetzt werden.

EP-B 0 913 388 betrifft ein Verfahren zum katalytischen Hydrieren von Nitrilen, das den Kontakt des Nitrils mit Wasserstoff in Gegenwart eines Kobaltschwammkatalysators bei Bedingungen für die Durchführung der Umwandlung der Nitrilgruppe in das primäre Amin umfasst. Der Kobaltschwammkatalysator ist zuvor mit einer katalytischen Menge von Lithiumhydroxid behandelt worden und das Verfahren wird in Gegenwart von Wasser durchgeführt. Als Nitrile eignen sich aliphatische Nitrile mit 1 bis 30 Kohlenwasserstoffatomen, unter anderem auch β-Aminonitrile wie Dimethylaminopropionitril. Ein weiteres Verfahren zur Herstellung von Polyaminen aus den entsprechenden Polynitrilen wird in DE-A 27 55 687 offenbart. In diesem Verfahren wird die Hydrierung an einem Hydrierungskatalysator in Tablettenform in Gegenwart eines den Zerfall des Katalysators inhibierenden Stabilisators durchgeführt. Als Polynitril kann unter anderem Ethylendiamindipropionitril (EDDPN) verwendet werden. Als Stabilisator eignet sich unter anderem EDA.

US-A 2006/0041170 betrifft ein Verfahren zur Herstellung von TETA, insbesondere von TETA-Salzen, und deren Verwendung als Arzneimittel. In diesem mehrstufigen Verfahren wird zunächst EDDN hergestellt. Anschließend wird EDDN mit Benzaldehyd unter Ausbildung eines (cyclischen) Imidazolidin-Derivates umgesetzt. Diese cyclische Verbindung, die zwei Cyano-Gruppen aufweist wird reduziert, beispielsweise durch Umsetzung mit Wasserstoff, wobei die entsprechende cyclische Diamino-Verbindung erhalten wird. Diese Diamino-Verbindung wird wiederum in Gegenwart einer Säure hydrolysiert unter Erhalt des entsprechenden TETA-Salzes. In einer alternativen Ausführungsform wird die cyclische Diamino-Verbindung ebenfalls mit Benzaldehyd umgesetzt unter Ausbildung der entsprechenden Diimin-Verbindung, die anschließend wiederum in Gegenwart einer Säure unter Erhalt des entsprechenden TETA-Salzes hydrolysiert wird. Als weitere Verfahrensalternative wird in diesem Dokument die Umsetzung von EDDN mit Boc-Schutzgruppen (Tertiär-Butoxy-Carbonyl-Gruppen) beschrieben. Das dabei erhaltene, zweifach mit Boc-Schutzgruppen geschützte EDDN-Derivat wird anschließend unter Erhalt des entsprechenden geschützten TETA-Derivates hydriert. Die Entfernung der Boc-Schutzgruppen erfolgt durch saure Hydrolyse unter Erhalt des entsprechenden TETA-Salzes. Nachteilig an diesem in US-A 2006/0041170 beschriebenen Verfahren ist insbesondere, dass es sich um ein mehrstufiges Hydrierverfahren handelt, bei dem das eingesetzte Edukt EDDN zuerst chemisch derivatisiert werden muss, um die Hydrierung durchzuführen. Weiterhin ist nachteilig, dass zunächst TETA als Salz und nicht in der freien Basenform anfällt.

Im Stand der Technik wird somit nirgendwo beschrieben, dass EDDN oder Aminonitrilgemische enthaltend EDDN und EDMN durch direkte Hydrierung des Aminonitrils zur Herstellung von TETA und gegebenenfalls weiterer Ethylenamine eingesetzt werden können. Andere (großtechnische) Verfahren zur Herstellung von TETA sind jedoch bekannt.

EP-A 222 934 betrifft ein Verfahren zur Herstellung von höheren Alkylenpolyaminen durch Umsetzung eines vicinalen Dihaloalkans mit einem Überschuss an Ammoniak in wässrige Phase unter Zugabe einer starken Base, wobei ein Imin-Zwischenprodukt gebildet wird, das anschließend mit einem Alkylenpolyamin unter Ausbildung des höheren Alkylenpolyamins umgesetzt wird. Als vicinales Dihaloalkan eignet sich insbesondere Ethylendichlorid (EDC oder 1,2-Dichlorethan). Als Alkylenpolyamine werden insbesondere Ethylendiamin oder höhere Ethylenamine wie DETA, aber auch TETA und Tetraethylenpentaamin (TEPA) eingesetzt. Bei diesen Verfahren (EDC-Verfahren) fällt ein Gemisch verschiedener Ethylenamine (lineare Ethylenamine wie EDA, DETA, TETA, TEPA oder höhere Ethylenamine sowie cyclische Derivate wie Piperazin (Pip) oder Aminoethyl-Piperazin (AEPip)) an. Je nachdem, welches Ethylenamin zu den Edukten EDC und NH₃ zugegeben wird, enthält das Reaktionsgemisch einen entsprechenden Anteil an höheren Ethylenaminen. Wenn beispielsweise gezielt TEPA hergestellt werden soll, wird den Edukten EDC und NH₃ das Ethylenamin TETA zugegeben. Auf diese Weise enthält das Produkt (Ethylenamingemisch) einen höheren Anteil an TEPA, jedoch auch die vorgenannten weiteren linearen sowie cyclischen Ethylenamine. Nachteilig an diesem Verfahren ist insbesondere, dass das Verfahren mit einer geringen Selektivität abläuft (bezüglich die Komponenten des erhaltenen Ethylenamingemisches) und dass zuerst ein spezielles Ethylenamin hergestellt werden muss (beispielsweise DETA), welches anschließend in das Verfahren eingebracht wird, um das nächst höhere Ethylenamin (beispielsweise TETA) gezielt herzustellen bzw. die Ausbeute zu erhöhen. Dieses Verfahren stellt außerdem aufgrund der eingesetzten Edukte (Halogenalkane) sowie der anfallenden Salzsäure ein Korrosionsproblem sowie aufgrund der anfallenden Salze ein Umweltproblem dar.

US-A 3,462,493 betrifft ein Verfahren zur Herstellung von TETA, wobei ein mindestens fünffach molarer Überschuss an EDA mit Ethylendichlorid oder Ethylendibromid umgesetzt wird. Als Nebenprodukte treten hierbei insbesondere Pip oder Piperazinoethylethylendiamin auf.

DE-T 689 11 508 beschreibt ein alternatives Verfahren zur Herstellung von linear verlängerter Polyalkylenpolyamine wie TETA. In diesem Verfahren wird ein bifunktioneller aliphatischer Alkohol mit einem Aminreaktanden in Anwesenheit eines Wolfram-haltigen Katalysators umgesetzt. Als bifunktioneller aliphatischer Alkohol eignet sich insbesondere Monoethanolamin (MEA), als Aminreaktanden können beispielsweise EDA oder DETA eingesetzt werden. Durch dieses Verfahren werden prinzipiell Gemische aus linear verlängerten Polyalkylenpolyaminen (also Ethylenamingemische) erhalten. In diesen Ethylenamingemischen ist an Ethylenaminen DETA, TETA, TEPA, Pip, AEPip oder Piperazinderivate höherer Ethylenamine enthalten, wobei der Anteil der jeweiligen Komponente je nach eingesetzten Aminreaktanden variiert. Sofern als Aminreaktand DETA verwendet wird, wird ein Ethylenamingemisch mit hohem Anteil an TETA und TEPA erhalten. An diesem Verfahren ist nachteilig, dass das Verfahren mit einer geringen Selektivität abläuft (bezüglich der Komponenten des erhaltenen Ethylenamingemisches) und dass zuerst ein zusätzliches Ethylenamin synthetisiert werden muss, das mit dem bifunktionalen aliphatischen Alkohol (beispielsweise MEA) umgesetzt wird. Hierbei entstehen in größerer Menge Nebenprodukte wie Aminoethylethanolamin (AEEA) oder höhere Hydroxy-haltige Ethylenamine, die von geringerem wirtschaftlichem Interesse sind. Die größere Menge an anfallenden Nebenprodukten ist darin begründet, da MEA bzw. die höheren Ethanolamine (z.B. AEEA) anstelle mit dem eingesetzten Amin mit sich selbst reagieren können. Aufgrund der (statistisch) vielen Reaktionsmöglichkeiten ist die Selektivität zum linearen TETA wegen der Koppelprodukte recht gering und nicht steuerbar. Die Synthese ist nur bei Teilumsatz möglich.

Einen Überblick über die Herstellung von Ethylenaminen liefert der SRI-Report "CEH Product Review Ethyleneamines"; SRI International, 2003; S. 53-54, in dem entsprechend der vorstehend beschriebenen Verfahren (mit den Edukten EDC oder MEA) insbesondere EDA oder DETA hergestellt werden. Dabei fallen höhere Ethylenamine wie TETA oder TEPA als Nebenprodukte an bzw. werden durch erneute Umsetzung der Edukte mit EDA oder DETA in höherer Ausbeute erhalten.

Weiterhin sind vereinzelt bereits Verfahren zur Herstellung von EDDN oder EDMN in der Literatur beschrieben worden. So beschreibt K. Masuzawa et al., Bull. Chem. Soc. Japan, Band 41 (1968) Seiten 702-706 ein Verfahren zur Herstellung und Reaktion von Stickstoff- und Schwefel-Analoga von 2-Piperazinon-Derivaten. Zur Herstellung dieser Stoffklasse wird zunächst von den Edukten EDA und FACH ausgegangen. Die Umsetzung der beiden Edukte erfolgt im äquimolaren Verhältnis, wobei Methanol als Lösungsmittel verwendet wird. Nachdem bei Raumtemperatur die Reaktionslösung zwei Tage lang stehengelassen wurde, wird nach Entfernung des Lösungsmittels sowie der nicht umgesetzten Edukte unter reduziertem Druck ein ölartiges Produkt erhalten. Dieses ölartige Produkt enthält neben einer cyclischen Verbindung auch EDMN als Nebenkomponente. Die Reaktion wurde unter Ausschluss von Wasser durchgeführt. Das ölartige Produkt wird anschließend in einem mehrstufigen Verfahren zu den gewünschten 2-Piperazinon-Derivaten weiter umgesetzt. Als unerwünschte Nebenreaktion bei der Umsetzung von EDA mit FACH wird in diesem Dokument auch die Herstellung von EDDN beschrieben. EDDN wird erhalten, sofern ein molarer Überschuss an EDA mit FACH in Methanol bei 55 bis 60°C umgesetzt wird. Nach Aufkonzentrierung unter reduziertem Druck erfolgt die Produktisolierung durch Vakuumdestillation. Hierbei wird eine Ausbeute von ca. 27,3 % bezogen auf das eingesetzte EDA erhalten.

H. Baganz et al., Chem. Ber, 90 (1957), Seiten 2944-2949 beschreiben ein Verfahren zur Darstellung von N,N'-Ethylen-bis-aminosäurederivaten, wobei das Dihydrochlorid von EDDN als Edukt dieses mehrstufigen Verfahrens dient. In diesem Dokument befindet sich auch eine Synthesevorschrift für das Dihydrochlorid von EDDN. Hierbei werden das Dihydrochlorid von EDA und Kaliumcyanid (KCN) in einem Reaktionsgefäß vorgelegt und anschließend 30 %iges Formaldehyd in das Reaktionsgefäß zugetropft, wobei die Reaktionstemperatur 25°C nicht übersteigt. Nach 12-stündiger Reaktionszeit und Zugabe von Natronlauge wird das Produkt mit Ether ausgeschüttelt, getrocknet, und durch Zugabe von Chlorwasserstoff als Ammoniumsalz ausgefällt. Das erhaltene Produkt wird anschließend kristallisiert. Nachteilig an diesem Verfahren ist insbesondere der Einsatz von Chlorwasserstoff sowie KCN, das die wässrige Phase sehr salzhaltig macht. Weiterhin ist die Extraktion mit Ether problematisch, da aufgrund der guten Wasserlöslichkeit von EDDN das Reaktionsprodukt nicht vollständig in die Etherphase gelangt.

H. Brown et al., Helvetica Chimica Acta, Band 43 (1960), Seiten 659-666 beschreiben ein Verfahren zur Herstellung von Komplexbildner aus der Thiazolreihe. In diesem mehrstufigen Verfahren wird EDDN als Ausgangsmaterial verwendet, weiterhin ist eine Synthesevorschrift zur Herstellung von EDDN in diesem Dokument enthalten. Gemäß dem darin beschriebenen Verfahren wird EDA in Wasser in einer Reaktionsvorrichtung vorgelegt, worauf unter Rühren und Eiskühlung gleichzeitig HCN sowie Kalziumcyanid (Ca(CN)₂) in Wasser zugegeben werden. In diesem Verfahren wird jedoch kein Formaldehyd verwendet. Nach aufwändiger Aufarbeitung wird in relativ geringer Ausbeute EDDN erhalten.

Die vorstehend bereits erwähnte US-A 2006/0041170 enthält ebenfalls Vorschriften zur Herstellung des darin beschriebenen Edukts EDDN. Einerseits kann EDDN durch direkte Alkylierung von EDA mit einem Haloacetonitril wie Chlor- oder Bromacetonitril hergestellt werden. Andererseits wird EDDN durch die bereits vorstehend beschriebene Umsetzung von EDA, insbesondere das Dihydrochlorid von EDA, zunächst mit Formaldehyd und anschließend mit Cyanosalzen wie KCN hergestellt. Das dabei erhaltene Reaktionsprodukt wird noch mit einer Säure behandelt. Sofern EDA als Edukt verwendet wird, erfolgt zunächst zusätzlich eine Überführung von EDA in dessen Salzform, insbesondere in Dihydrochlorid, bevor mit Formaldehyd weiter umgesetzt wird. Nachteilig an diesem Verfahren ist der Salzanfall durch die Verwendung von Cyanosalzen. Ebenso nachteilig ist das Feststoff-Handling von EDA-Salzen, da EDA entweder direkt als Salz eingesetzt oder zunächst in ein Salz überführt wird. Außerdem eignen sich das in US-A 2006/0041170 beschriebene Verfahren nicht zur kontinuierlichen Anwendung.

Aufgabe der vorliegenden Erfindung ist es demnach, ein einfaches und kostengünstiges Verfahren zur Herstellung von TETA und gegebenenfalls in gezielten Mengen weitere Ethylenamine bereitzustellen.

Die Aufgabe wird gelöst durch ein Verfahren zur Herstellung von Triethylentetraamin (TETA) umfassend die folgenden Schritte:
a) Ethylendiamin (EDA) wird mit Formaldehyd und Blausäure (HCN) umgesetzt, wobei das molare Verhältnis von EDA zu Formaldehyd zu HCN 1 : 1,5 : 1,5 bis 1 : 2 : 2 [mol/mol/mol] beträgt, unter Erhalt von Ethylendiamindiacetonitril (EDDN),
b) Hydrierung des in Schritt a) erhaltenen EDDN's in Gegenwart eines Katalysators und eines Lösungsmittels.

Das erfindungsgemäße Verfahren hat den Vorteil, dass TETA und gegebenenfalls die weitere Hauptkomponente DETA bei hohem Umsatz und/oder Selektivität hergestellt werden können Die erhöhte Selektivität zeigt sich insbesondere darin, dass das eingesetzte EDDN überwiegend zu TETA hydriert wird. Die dabei gebildeten Nebenprodukte sind hauptsächlich weitere lineare Ethylenamine. Der Anteil an cyclischen Ethylenaminen ist im erfindungsgemäßen Verfahren relativ gering. Die weiteren Ethylenamine sind jedoch teilweise ebenfalls interessante Wertprodukte (hauptsächlich die linearen Ethylenamine wie DETA), deren Isolierung beispielsweise in großtechnischen Verfahren lohnenswert ist. Cyclische Ethylenamine wie AEPip sind hingegen als Wertprodukt von eher geringem Interesse, können allerdings durch Rückführung weiter umgesetzt werden.

Die Selektivität des erfindungsgemäßen Verfahrens kann in vorteilhafter Weise dadurch erhöht werden, dass vor der Hydrierung eine Leichtsiederabtrennung insbesondere von Blausäure (HCN) durchgeführt wird. Die Blausäure kann dabei auch als Zersetzungsprodukt von Formaldehydcyanhydrin (FACH) auftreten. Insbesondere Blausäure kann als Katalysatorgift bei der Hydrierung wirken.

Sofern die Leichtsieder abgetrennt worden sind, können EDDN sowie gegebenenfalls weitere Aminonitrile wie EDMN oder DETDN schneller und in höherer Selektivität hergestellt werden. Dies wirkt sich auch positiv auf die Selektivität in der anschließenden Hydrierung aus.

In vorteilhafter Weise wird EDDN und gegebenenfalls EDMN vollständig oder nahezu vollständig umgesetzt. Dies ist insbesondere bei großtechnischen Verfahren von Bedeutung, da nicht umgesetztes Edukt in der Regel in den Produktionskreislauf zurückgeführt wird bzw. entsorgt werden muss. Verfahren, bei denen größere Mengen an EDDN und/oder EDMN nicht umgesetzt werden, sind aufgrund der hohen Instabilität des EDDN bzw. EDMN von besonderem Nachteil. Einerseits neigen sowohl EDDN als auch EDMN sich bei höheren Temperaturen zu zersetzen, so dass die Zersetzungsprodukte nicht in den jeweiligen Kreislauf zurückgeführt werden können, andererseits kann diese Zersetzung auch mit explosionsartiger Heftigkeit verlaufen. Da im erfindungsgemäßen Verfahren die Aminonitrile vollständig umgesetzt werden können, müssen hinsichtlich der Rückführung in den Produktionszyklus keine Anstrengungen unternommen werden.

Ein weiterer Vorteil des erfindungsgemäßen Verfahrens ist, dass im Gegensatz zum EDC-Verfahren auf den Einsatz von chlorierten Kohlenwasserstoffen als Edukt verzichtet werden kann. Zudem fällt keine Salzsäure bzw. deren Salze als weiteres Reaktionsprodukt an. Die Entsorgung der vorgenannten Stoffe ist insbesondere bei großtechnischen Verfahren ein (Umwelt-)Problem. Vorteilhaft im Vergleich zum MEA-Verfahren ist, dass aufgrund der unterschiedlichen Edukte die Bildung von AEEA sowie weiterer Verbindungen mit Hydroxy-Funktion keine Rolle spielt. Ebenso ist es ein Vorteil, dass das erfindungsgemäße Verfahren kontinuierlich durchgeführt werden kann.

Sofern im erfindungsgemäßen Verfahren ein Aminonitrilgemisch hydriert wird, ist es als Vorteil anzusehen, dass, je nach Bedürfnissen des Marktes, ein höherer oder niedriger Anteil an TETA oder DETA hergestellt werden kann. Dies ist darin begründet, dass sich prinzipiell das Verhältnis der Edukte EDMN zu EDDN im Produkt hinsichtlich DETA zu TETA wieder findet. So können im erfindungsgemäßen Verfahren spezielle Aminonitrilgemisch-Zusammensetzungen gezielt eingesetzt werden, um die im Markt gewünschten Mengenverhältnisse zu bedienen. Das erfindungsgemäße Verfahren liefert bei hoher Selektivität ein Ethylenamingemisch, das mindestens 30 % TETA, neben mindestens 5 % DETA sowie gegebenenfalls weitere Ethylenamine wie Piperazinderivate als Wertprodukte erhält.

Das erfindungsgemäße Verfahren hat weiterhin aufgrund von Schritt a) den Vorteil, dass EDDN sowie gegebenenfalls ein gezieltes Gemisch enthaltend EDDN und weitere Aminonitrile wie EDMN in einfacher Weise sowie in hoher Reinheit hergestellt werden können. Sowohl EDDN als auch gegebenenfalls EDMN können auf einfache Weise in Form von reinen Kristallen isoliert werden, während hingegen in den Verfahren gemäß dem Stand der Technik das Produkt als viskose Flüssigkeit anfällt, aus der das gewünschte Produkt nur durch aufwändige Aufarbeitungsschritte und in schlechter Reinheit isoliert werden kann.

Ein weiterer Vorteil des erfindungsgemäßen Verfahrens ist, dass auf Cyanosalze wie KCN und Ca(CN)₂ verzichtet werden kann. Zudem wird im erfindungsgemäßen Verfahren in vorteilhafter Weise EDA nicht als Salz, sondern in Form der freien Base als Edukt eingesetzt. Im erfindungsgemäßen Verfahren ist folglich der Anteil an Salzen in der Reaktionslösung geringer, und es fallen somit auch weniger salzhaltige Nebenprodukte bzw. nicht-umgesetzte Edukte an. Dies ist insbesondere bei großtechnischen Verfahren von Vorteil. Ein hoher Salzgehalt in der Reaktionslösung ist insbesondere dann von Nachteil, wenn das erfindungsgemäße Verfahren in wässriger Phase durchgeführt wird. Folglich kann mit weniger Reaktionsschritten das gewünschte Produkt in einfacher Weise hergestellt werden. Weiterhin ist es vorteilhaft, dass das erfindungsgemäße Verfahren, insbesondere Schritt a), kontinuierlich durchgeführt werden kann.

Ein weiterer wesentlicher Vorteil des erfindungsgemäßen Verfahrens ist darin zu sehen, dass ausgehend von nur einem Edukt (EDA) ein Ethylenamingemisch hergestellt werden kann, das zwei (Haupt-)Produkte (TETA und DETA) in gezielt einstellbaren Verhältnissen zueinander enthält. In einer weiteren Ausführungsform der vorliegenden Erfindung kann das im Ethylengemisch enthaltene DETA ganz oder teilweise nach Schritt a) zurückgeleitet werden, um es vorzugsweise mit FACH umzusetzen. Auf diese Weise werden DETDN und/oder DETMN hergestellt, die anschließend zu TEPA beziehungsweise TETA weiter hydriert werden. Folglich können durch das erfindungsgemäße Verfahren ausgehend von einem Edukt (EDA) sowie teilweise Rückführung des Hydrierungsproduktes DETA die unterschiedlichen Ethylenamine TEPA, TETA und DETA hergestellt werden. Je nach den gewählten Ausgangsbedingungen können Ethylenamingemische hergestellt werden, in denen die vorgenannten Hauptprodukte in variablen Verhältnissen zueinander vorliegen, wobei die einzelnen Ethylenamine (TETA, TEPA und/oder DETA sowie gegebenenfalls weitere Nebenprodukte) aus dem Ethylenamingemisch isoliert werden können.

In einer weiteren Ausführungsform ist auch die Rückführung nach Schritt a) des bei der Hydrierung als cyclisches Nebenprodukt gebildeten AEPip denkbar. Die Umsetzung von AEPip zu den entsprechenden Aminonitrilen und anschließender Hydrierung liefert Diaminoethylpiperazin (DAEPip), Piperazinethylethylendiamin (PEEDA) sowie Aminoethylpiperazinylethylethylendiamin (AEPEEDA). Diese cyclischen Ethylenamine sind ebenfalls als Wertprodukt von Interesse.

### Schritt a)

Gemäß Schritt a) wird Ethylendiamin (EDA) mit Formaldehyd und Blausäure (HCN) umgesetzt, wobei das molare Verhältnis von EDA zu Formaldehyd zu HCN 1 : 1,5 : 1,5 bis 1 : 2 : 2 [mol/mol/mol] beträgt, unter Erhalt von Ethylendiamindiacetonitril (EDDN).

Sofern nachstehend nicht anders angegeben (Option a1) bis a4)), können die Eduktkomponenten von Schritt a) in beliebiger Reihenfolge in das jeweilige Reaktionsgefäß gegeben werden. Beispielsweise kann ein Edukt vollständig vorgelegt werden und ein zweites Edukt zugegeben werden. Vorzugsweise kann EDDN gemäß einer der nachfolgend aufgeführten Optionen a1) bis a4) hergestellt werden. Besonders bevorzugt wird EDDN nach Option a1) hergestellt.

Gemäß Option a1) wird Formaldehyd und HCN zunächst zu Formaldehydcyanhydrin (FACH) umgesetzt, wobei anschließend EDA wiederum mit FACH umgesetzt wird und das molare Verhältnis von EDA zu FACH 1 : 1,5 bis 1 : 2 [mol/mol] beträgt. EDA, Formaldehyd und HCN sind kommerziell erhältliche Produkte oder können prinzipiell nach dem Fachmann bekannten Methoden hergestellt werden. Vorzugsweise wird EDA im erfindungsgemäßen Verfahren in Form seiner freien Base eingesetzt, gegebenenfalls können jedoch auch Salze wie das Dihydrochlorid von EDA als Edukt verwendet werden.

Die Umsetzung von Formaldehyd und HCN ist dem Fachmann bekannt. Die Herstellung von FACH kann durch Umsetzung von wässrigem Formaldehyd mit Blausäure erfolgen. Vorzugsweise liegt Formaldehyd als 30 bis 50 %ige wässrige Lösung vor, Blausäure wird vorzugsweise in 90 bis 100 %iger Reinheit eingesetzt. Diese Umsetzung erfolgt vorzugsweise bei einem pH-Wert von 5,5, der vorzugsweise mit Natronlauge oder Ammoniak eingestellt wird. Die Umsetzung kann bei Temperaturen von 20 bis 70°C beispielsweise im Schlaufen- und/oder Rohrreaktor erfolgen.

Anstelle von aufgereinigter Blausäure (HCN) kann auch HCN-Rohgas in wässriger Formaldehyd-Lösung unter den oben genannten Bedingungen zu FACH chemisobiert werden. Das HCN-Rohgas wird vorzugsweise durch Pyrolyse von Formamid hergestellt und enthält neben Wasser insbesondere geringe Anteile an Ammoniak.

Gegebenenfalls kann die erhaltene wässrige FACH-Lösung durch schonende Vakuumeindämpfung, beispielsweise mit einem Fallfilm- oder Dünnschichtverdampfer, aufkonzentriert und von Leichtsiedern, insbesondere von Blausäure befreit werden. Vorzugsweise erfolgt eine Aufkonzentrierung auf eine 50-80 %ige FACH-Lösung. Vor der Aufkonzentrierung ist eine Stabilisierung der FACH-Lösung durch Erniedrigung des pH-Wertes auf ≤ 4, bevorzugt auf ≤ 3 vorteilhaft, beispielsweise durch Säurezugabe, zum Beispiel durch Zugabe von Phosphorsäure oder vorzugsweise von Schwefelsäure.

Vorzugsweise beträgt in Option a1) das molare Verhältnis von EDA zu FACH ungefähr 1 : 1,8 bis 1 : 2 [mol/mol], insbesondere ca. 1 : 2 [mol/mol].

Nach Option a2) wird EDDN durch Umsetzung eines Ethylendiamin-Formaldehyd-Addukt (EDFA) mit Blausäure (HCN) umgesetzt, wobei das molare Verhältnis von EDFA zu HCN 1 : 1,5 bis 1 : 2 [mol/mol] beträgt. Vorzugsweise beträgt das molare Verhältnis von EDFA zu HCN 1 : 1,8 bis 1 : 2 [mol/mol], insbesondere ca. 1 : 2 [mol/mol]. EDFA wird vorzugsweise durch Vermischen von in etwa äquimolaren Mengen an EDA und Formaldehyd hergestellt.

Gemäß Option a3) wird EDA mit einem Gemisch aus Formaldehyd und Blausäure (GFB) umgesetzt, wobei das molare Verhältnis von EDA zu GFB 1 : 1,5 bis 1 : 2 [mol/mol] beträgt. Vorzugsweise beträgt das molare Verhältnis von EDA zu GFB 1 : 1,8 bis 1 : 2 [mol/mol], insbesondere ca. 1 : 2 [mol/mol]. Vorzugsweise wird das GFB durch Vermischen von in etwa äquimolaren Mengen aus Formaldehyd und Blausäure hergestellt.

Gemäß Option a4) wird EDA mit Formaldehyd und Blausäure (HCN) zeitlich (parallel) umgesetzt, wobei das molare Verhältnis von EDA zu Formaldehyd zu HCN 1 : 1,5 : 1,5 bis 1 : 2 : 2 [mol/mol/mol] beträgt. Vorzugsweise beträgt das molare Verhältnis von EDA zu Formaldehyd zu HCN 1 : 1,8 : 1,8 bis 1 : 2 : 2 [mol/mol/mol], insbesondere ca. 1 : 2 : 2 [mol/mol/mol]. Vorzugsweise werden in dieser Ausführungsform die drei Eduktkomponenten zeitgleich oder schrittweise in gleichen molaren Teilmengen bezogen auf die jeweilige Eduktgesamtmenge in das Reaktionsgefäß zugegeben.

Unter Umständen können die jeweiligen Edukte beziehungsweise Zwischenprodukte direkt im Anschluss an ihre Herstellung im erfindungsgemäßen Verfahren eingesetzt werden. Beispielsweise kann in Option a1) FACH ohne vorherige Isolierung im erfindungsgemäßen Verfahren als Edukt eingesetzt werden. Gegebenenfalls kann jedoch FACH im Anschluss an dessen Herstellung zuerst isoliert werden, um anschließend im erfindungsgemäßen Verfahren eingesetzt zu werden.

In einer Ausführungsform der vorliegenden Erfindung wird Schritt a) frei oder zumindest im Wesentlichen frei von Cyanosalzen, wie KCN, durchgeführt.

Schritt a) des erfindungsgemäßen Verfahrens wird normalerweise in Gegenwart eines Lösungsmittels durchgeführt. Vorzugsweise werden im erfindungsgemäßen Verfahren zur Herstellung von EDDN die Edukte in wässriger Phase umgesetzt. Gegebenenfalls können neben Wasser noch weitere, dem Fachmann bekannte Lösungsmittel verwendet werden, die mit Wasser mischbar sind. Weniger bevorzugt werden jedoch Alkohole, insbesondere Methanol als Lösungsmittel verwendet.

Schritt a) wird vorzugsweise bei einer Temperatur von 10 bis 90°C, insbesondere bei 30 bis 70°C durchgeführt. Die Reaktion kann bei Normaldruck oder gegebenenfalls auch bei erhöhtem Druck (Überdruck) durchgeführt werden. Vorzugsweise wird Schritt a) in einem Rohrreaktor oder einer Rührkesselkaskade durchgeführt. Vorzugsweise kann Schritt a) auch als kontinuierliches Verfahren durchgeführt werden, insbesondere als großtechnisches Verfahren.

Neben dem Hauptprodukt EDDN fällt in Schritt a) des erfindungsgemäßen Verfahrens als wichtigstes Nebenprodukt Ethylendiaminmonoacetonitril (EDMN) an. Je nach Wahl der entsprechenden Verfahrensparameter (beispielsweise Edukt, Temperatur, Lösungsmittel oder Druck) kann das erfindungsgemäße Verfahren so gesteuert werden, dass der Anteil an EDMN im Reaktionsprodukt variiert und EDMN nicht als Nebenprodukt sondern als zweites Reaktionshauptprodukt anfällt. In dieser Ausführungsform der vorliegenden Erfindung wird somit ein Aminonitrilgemisch hergestellt, das neben EDDN auch EDMN (als Hauptprodukt) enthält. Vorzugsweise werden dabei Aminonitrilgemische hergestellt, enthaltend mindestens 30 Gew.-% EDDN und mindestens 5 Gew.-% EDMN. EDDN ist normalerweise zu 30 bis 95 Gew.-%, bevorzugt zu 50 bis 95 Gew.-%, besonders bevorzugt zu 75 bis 90 Gew.-% im Aminonitrilgemisch enthalten. Das Aminonitrilgemisch enthält EDMN normalerweise zu 5 bis 70 Gew.-%, bevorzugt zu 5 bis 50 Gew.-%, besonders bevorzugt zu 10 bis 25 Gew.-%. Die vorstehenden Werte beziehen sich nur auf das Verhältnis EDDN zu EDMN ohne Berücksichtigung weiterer Aminonitrile, die durch Rückführung von Hydrierungsprodukten aus Schritt b) gebildet worden sind.

Die vorstehenden Gewichtsprozentangaben von EDDN beziehungsweise EDMN beziehen sich auf die Gesamtmenge der im Gemisch enthaltenen Aminonitrile. Zusätzlich vorhandenes Wasser oder sonstige Lösungsmittel sind bei diesen Mengenangaben nicht berücksichtigt.

Vorzugsweise wird eine Erhöhung des Anteils an EDMN im Aminonitrilgemisch dadurch erzielt, dass in den Optionen a1) - a4) in den angegebenen Parameterbereichen jeweils ein geringerer molarer Anteil an FACH (Option a1)), HCN (Option a2)), GFB (Option a3)) oder Formaldehyd und HCN (Option a4)) verwendet wird. So wird beispielsweise gemäß Option a) zur Erhöhung des EDMN-Anteils ein molares Verhältnis von EDA zu FACH von 1 : 1,5 bis 1 : 1,8 [mol/mol] verwendet. Weiterhin kann zur Herstellung eines Aminonitrilgemisches, das einen geringeren Anteil an EDMN enthält, beispielsweise ≤ 10 Gew.-%, insbesondere 5 bis 10 Gew.-%, in einer Ausführungsform der vorliegenden Erfindung durch Umsetzung von EDA mit einem möglichst hohen molaren Anteil an FACH hergestellt werden. Hierbei wird vorzugsweise eine wässrige Lösung ≥ 40 Gew.-% an FACH oder reines FACH eingesetzt. Das molare Verhältnis von EDA zu FACH beträgt in diesem Fall vorzugsweise 1 : 2 [mol/mol].

Gegebenenfalls kann gemäß der vorliegenden Erfindung auch sehr reines EDDN mit einem geringen Anteil an EDMN hergestellt werden. Vorzugsweise beträgt der Gehalt an EDMN sowie eventuell an noch weiteren Nebenprodukten, beispielsweise sonstige Aminonitrile, ≤ 10 Gew.-%, insbesondere ≤ 5 Gew.-%, bezogen auf EDDN.

### Schritt b)

Schritt b) umfasst die Hydrierung des in Schritt a) erhaltenen EDDN's in Gegenwart eines Katalysators und eines Lösungsmittels. Hydrieren im Rahmen der vorliegenden Erfindung bedeutet die Reaktion von EDDN und gegebenenfalls EDMN sowie eventuell vorhandener weiterer Aminonitrile mit Wasserstoff.

Schritt b) kann direkt im Anschluss an Schritt a) durchgeführt werden, gegebenenfalls können zwischen Schritt a) und Schritt b) eine oder mehrere (der nachfolgend aufgeführten Aufreinigungsschritte) durchgeführt werden.

### i) Leichtsieder-Abtrennung

In einer Ausführungsform der vorliegenden Erfindung werden vor der Hydrierung die Leichtsieder aus dem Reaktionsprodukt von Schritt a) abgetrennt. Sofern zur Herstellung von EDDN und gegebenenfalls EDMN FACH verwendet wird, kann die Leichtsiederabtrennung bereits vor der Umsetzung von FACH mit EDA erfolgen.

Vorzugsweise werden als Leichtsieder Blausäure (HCN) abgetrennt. HCN kann dabei auch als Zersetzungsprodukt von FACH auftreten. Weiterhin kann an dieser Stelle eventuell vorhandener Ammoniak abgetrennt werden. Vorzugsweise erfolgt die Abtrennung durch Destillation, beispielsweise in Form einer Dünnschichtverdampfung wie z.B. einer Sambay-Destillation ("Chemie Ingenieur Technik, Vol. 27, S. 257-261).Gegebenenfalls kann das Reaktionsgemisch auch mit Stickstoff gestrippt werden.

### ii) Abreicherung von Wasser

Wasser kann entweder bevorzugt zusammen mit den Leichtsiedern oder, nach der Leichtsiederabtrennung ganz oder teilweise abgereichert werden. Vorzugsweise erfolgt die Abreicherung des Wassers als Destillation. Dies kann ein- oder mehrstufig in einem Verdampfer bzw. einer Verdampferkaskade erfolgen, wobei von Stufe zu Stufe unterschiedliche Drücke bzw. Temperaturen eingestellt werden können. Die Wasserabtrennung kann auch in einer Destillationskolonne erfolgen. Bevorzugt erfolgt die Wasserabtrennung im Vakuum. Das verbleibende Aminonitril oder Aminonitrilgemisch kann noch Reste an Wasser und Leichtsieder enthalten. Bevorzugt ist ein Restwassergehalt von mindestens 10 Gew.-%. Die Leichtsieder sind dann nur noch in geringen Spuren enthalten. Denkbar ist auch, die Leichtsieder- und Wasserabtrennung nach der FACH-Synthese durchzuführen.

### iii) Adsorption von Verunreinigungen

Das im Schritt a) erhaltene Aminonitril(gemisch) kann entweder direkt oder nach erfolgter Leichtsiederabtrennung oder nach erfolgter Leichtsieder- und Wasserabtrennung durch Adsorption von Verunreinigungen an einem Adsorbens, z.B. Aktivkohle oder lonentauscher, gereinigt werden. Dies kann z.B. in einer mit dem Adsorbens gefüllten Adsorptionskolonne erfolgen.

EDDN ist bei Raumtemperatur ein Feststoff, ebenso EDMN. Folglich wird Schritt b) des erfindungsgemäßen Verfahrens in Gegenwart von einem Lösungsmittel wie einem organischen Lösungsmittel und/oder Wasser durchgeführt. Vorzugsweise wird Wasser als Lösungsmittel verwendet, gegebenenfalls können auch Gemische aus Wasser und organischen Lösungsmitteln wie Ether, insbesondere THF, verwendet werden. Die zusätzliche Verwendung eines organischen Lösungsmittels (inerten organischen Verbindung) neben Wasser erweist sich jedoch als vorteilhaft, da dadurch eine Stabilisierung der einzelnen Komponenten des wässrigen Aminonitrilgemisches, insbesondere in Gegenwart der resultierenden Amine, erzielt werden kann. Außerdem lässt sich durch die Verwendung von organischen Lösungsmitteln ein Spüleffekt (Reduzierung der Spülzyklen, Verminderung der Katalysatorausschleusung) an dem eingesetzten Katalysator erzielen, wodurch dessen Standzeit erhöht bzw. dessen Verbrauch erniedrigt (längere Katalysatorlebensdauer) sowie die Katalysatorbelastung verbessert werden kann. Durch die Verwendung geeigneter Lösungsmittel kann weiterhin die Bildung von weiteren Nebenprodukten wie AEPip vermindert werden.

Ein geeignetes Lösungsmittel, welches ein oder mehrere Komponenten umfassen kann, sollte bevorzugt folgende Eigenschaften aufweisen:
(a) das Lösungsmittel sollte stabilisierend auf EDDN oder gegebenenfalls EDMN wirken, insbesondere deren Zersetzung bei den herrschenden Temperaturen verhindern;
(b) das Lösungsmittel sollte eine gute Wasserstofflöslichkeit zeigen;
(c) das Lösungsmittel sollte bei den Reaktionsbedingungen inert sein;
(d) das Reaktionsgemisch (EDDN oder gegebenenfalls EDMN, gegebenenfalls Wasser sowie Lösungsmittel) sollte unter Reaktionsbedingungen einphasig sein;
(e) die Lösemittelauswahl sollte im Hinblick auf eine bevorzugt destillative Abtrennung des Produktes im Anschluss an die Hydrierung aus dem Produktstrom erfolgen. Wobei energie- oder apparativ-aufwändige (z.B. engsiedende Gemische oder schwierig zu trennende Azeotrope) Trennungen zu vermeiden sind.
(f) das Lösungsmittel sollte gut von den Produkten abtrennbar sein, d.h. die Siedetemperatur sollte sich hinreichend von der der Produkte unterscheiden. Hierbei wird eine niedrigere Siedetemperatur als die der Produkte bevorzugt.

Mögliche Lösungsmittel (neben Wasser) sind organische Lösungsmittel, beispielsweise Amide, wie N-Methylpyrrolidon (NMP) und Dimethylformamid (DMF), aromatische und aliphatische Kohlenwasserstoffe, wie Benzol und Xylol, Alkohole wie Methanol, Ethanol, Propanol, Isopropanol, Butanol, Isobutanol, sekundäres Butanol und tertiäres Butanol, Amine, Ester, wie Essigsäuremethylester oder Essigsäureethylester und Ether, wie Diisopropylether, Diisobutylether, Glykoldimethylether, Diglykoldimethylether, Dioxan und Tetrahydrofuran (THF). Bevorzugt werden im erfindungsgemäßen Verfahren Ether verwendet, mehr bevorzugt cyclische Ether und besonders bevorzugt Tetrahydrofuran. In einer weiteren bevorzugten Ausführungsform werden Alkohole, insbesondere Methanol, als organisches Lösungsmittel verwendet.

Das Lösungsmittel wird im Gewichtsverhältnis zu dem eingesetzten Aminonitril (EDDN und gegebenenfalls EDMN) von 0,1 : 1 bis 15 : 1 eingesetzt. Die Konzentration des Aminonitrilgemisches in der Lösung, in der die Hydrierung durchgeführt wird, sollte so gewählt werden, dass eine geeignete Zuführrate bzw. Verweilzeit eingestellt werden kann. Es ist bevorzugt, das Aminonitril zu 10 bis 50 Gew.-% mit dem Lösungsmittel zu vermischen. Bezogen auf die besonders bevorzugten Lösungsmittel Methanol bzw. Tetrahydrofuran ist es beispielsweise vorteilhaft, das Aminonitril zu 20 bis 40 Gew.-% bezogen auf das Lösungsmittel einzusetzen.

Sofern Wasser vorhanden ist, liegt der Anteil an Wasser in der Lösung in einem Bereich von 0 bis 60 Gew.-%, vorzugsweise bei 10 bis 30 Gew.-%. Die Mengenangaben des Wassers beziehen sich dabei auf das Aminonitrilgemisch.

Gegebenenfalls können in der Lösung, in der die Hydrierung durchgeführt wird, zusätzliche Additive enthalten sein. Als Additive kommen prinzipiell Hydroxide wie Alkalimetallhydroxide, Alkoholate, Amide, Amine in Frage. Vorzugsweise eignen sich als Additive Amine, besonders EDA und Ammoniak, insbesondere EDA. Weiterhin können auch saure Additive, wie zum Beispiel Silikate zusätzlich in der Lösung enthalten sein. Diese Substanzen können als Reinstoff oder gelöst in einem Lösungsmittel zugesetzt werden. Vorzugsweise wird das erfindungsgemäße Verfahren unter Zusatz von Additiven durchgeführt.

In einer Ausführungsform des Verfahrens wird der Lösung, in der die Hydrierung durchgeführt wird, kein Ammoniak zugesetzt. Sofern noch Ammoniak in den Edukten bzw. in der gegebenenfalls eingesetzten wässrigen Lösung gelöst ist bzw. als Nebenprodukt bei der Hydrierung freigesetzt wird, ist dies nicht störend. Gegebenenfalls vorhandener Ammoniak kann nach dem Fachmann bekannten Methoden, beispielsweise destillativ, entfernt werden. Sofern auf Ammoniak verzichtet wird, hat dies den Vorteil, dass der Eigendruck des Systems verringert ist.

Als Katalysatoren zur Hydrierung der Nitril-Funktion zum Amin können Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten. In einer bevorzugten Ausführungsform werden im erfindungsgemäßen Verfahren Raney-Katalysatoren eingesetzt, bevorzugt Raney-Kobalt- oder Raney-Nickel-Katalysatoren und besonders bevorzugt mit mindestens einem der Elemente Cr, Ni oder Fe dotierte Raney-Kobalt- oder mit einem der Elemente Mo, Cr oder Fe dotierte Raney-Nickel-Katalysatoren.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff enthaltendem Gasstrom bei erhöhter Temperatur aktiviert. Wenn die Katalysatoren außerhalb des Reaktors reduziert werden, kann danach eine Passivierung durch einen Sauerstoff enthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können. Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂,1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31,5 Gew.-% ZrO₂, 50 Gew.-% NiO, 17 Gew.-% CuO und 1,5 Gew.-% MoO₃. Ebenso geeignet sind die in WO-A 99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als 'Metallschwamm' aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach z. B. mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typischerweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

In einer bevorzugten Ausführungsform wird erfindungsgemäß ein Raney-Kobalt-Skelett-Katalysator eingesetzt, der aus einer Co/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni oder Cr enthält.

Solche Katalysatoren enthalten typischerweise neben Kobalt noch 1 bis 30 Gew.-% Al, besonders 2 bis 12 Gew.-% Al, ganz besonders 3 bis 6 Gew.-% Al, 0 bis 10 Gew.-% Cr, besonders 0,1 bis 7 Gew.-% Cr, ganz besonders 0,5 bis 5 Gew.-% Cr, insbesondere 1,5 bis 3,5 Gew.-% Cr, 0 bis 10 Gew.-% Fe, besonders 0,1 bis 3 Gew.-% Fe, ganz besonders 0,2 bis 1 Gew.-% Fe, und/oder 0 bis 10 Gew.-% Ni, besonders 0,1 bis 7 Gew.-% Ni, ganz besonders 0,5 bis 5 Gew.-% Ni, insbesondere 1 bis 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:
Al: 2 bis 6 Gew.-%, Co: ≥ 86 Gew.-%, Fe: 0 bis 1 Gew.-%, Ni: 1 bis 4 Gew.-%, Cr: 1,5 bis 3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Cr enthält.

Solche Katalysatoren enthalten typischerweise neben Nickel noch
1 bis 30 Gew.-% Al, besonders 2 bis 20 Gew.-% Al, ganz besonders 5 bis 14 Gew.-% Al,
0 bis 10 Gew.-% Cr, besonders 0,1 bis- 7 Gew.-% Cr, ganz besonders 1 bis 4 Gew.-% Cr,
   und/oder
0 bis 10 Gew.-% Fe, besonders 0,1 bis- 7 Gew.-% Fe, ganz besonders 1 bis 4 Gew.-% Fe,
   wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden. Dieser Katalysator weist folgende Zusammensetzung auf
Al: ≤ 14 Gew.-%, Ni: ≥ 80 Gew.-%, Fe: 1 bis 4 Gew.-%, Cr: 1 bis 4 Gew.-%.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

Die Temperaturen, bei denen Schritt b) durchgeführt wird, liegen in einem Bereich von 40 bis 150°C, bevorzugt von 70 bis 140°C, insbesondere bei 80 bis 140°C.

Der bei der Hydrierung herrschende Druck liegt im Allgemeinen bei 5 bis 300 bar, bevorzugt bei 30 bis 250 bar, besonders bevorzugt bei 40 bis 160 bar

In einer bevorzugten Ausführungsform wird EDDN beziehungsweise das Aminonitrilgemisch enthaltend EDDN mit einer Rate der Hydrierung zugeführt, die nicht größer ist als die Rate, mit der EDDN und gegebenenfalls die übrigen Komponenten des Aminonitrilgemisches mit Wasserstoff bei der Hydrierung reagiert.

Die Zuführrate ist bevorzugt so einzustellen, dass quasi Vollumsatz erreicht wird. Dieses wird durch Temperatur, Druck, Art des Gemisches, Menge und Art des Katalysators, des Reaktionsmediums, Durchmischungsgüte des Reaktorinhalts, Verweilzeit etc. beeinflusst.

Im erfindungsgemäßen Verfahren werden ein (oder mehrere) Lösungsmittel verwendet, wobei das Lösungsmittel zunächst mit EDDN oder dem Aminonitrilgemisch vermischt wird. Die erhaltene Lösung, die gegebenenfalls auch Additive enthalten kann, wird anschließend in das den Katalysator enthaltende Reaktionsgefäß zugeführt. Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird. Bei kontinuierlichen Verfahren kann eine Teilmenge des Lösungsmittels auch separat von der Lösung, die EDDN, das Lösungsmittel und gegebenenfalls das Additiv enthält, in das Reaktionsgefäß zugegeben werden. In einer bevorzugten Ausführungsform erfolgt die Zuführung des in der Lösung enthaltenen EDDNs sowie der gegebenenfalls enthaltenen weiteren Aminonitrile wie EDMN mit einer Rate, die nicht größer ist als die Rate, mit der EDDN mit Wasserstoff bei der Hydrierung reagiert. Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird.

Das erfindungsgemäße Verfahren zur Herstellung von TETA durch Hydrierung von EDDN kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des Aminonitrils und des Katalysators mit dem gasförmigen Wasserstoff unter Druck möglich ist.

Die Hydrierung in Suspensionsfahrweise kann in einem Rührreaktor, Strahlschlaufenreaktor, Strahldüsenreaktor, Blasensäulenreaktor, bzw. in einer Kaskade derartiger gleicher oder verschiedener Reaktoren durchgeführt werden. Für die Hydrierung an einem Festbettkatalysator sind Rohreaktoren aber auch Rohrbündelreaktoren denkbar.

Im Fall eines Festbettkatalysators wird in Sumpf- oder Rieselfahrweise mit dem Aminonitril beaufschlagt. Bevorzugt wird allerdings die Suspensionsfahrweise in semikontinuierlicher und bevorzugt in kontinuierlicher Fahrweise eingesetzt.

Die Hydrierung der Nitrilgruppen findet unter Freisetzung von Wärme statt, die in der Regel abgeführt werden muss. Die Wärmeabfuhr kann durch eingebaute Wärmeüberträgerflächen, Kühlmantel oder außenliegende Wärmeüberträger in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen. Insbesondere kann der Kreislaufstrom mittels eines externen Wärmeübertragers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbetteinem Suspensionsreaktor nachgeschaltet.

Das erfindungsgemäße Verfahren liefert als Hauptprodukt das lineare Ethylenamin (C₆-Produkt) TETA (1. Fall) sowie weitere Ethylenamine als Nebenkomponenten. Sofern im erfindungsgemäßen Verfahren ein Aminonitrilgemisch enthaltend EDDN und EDMN eingesetzt wird, erhält man ein Ethylenamingemisch, das als Hauptkomponente die beiden linearen Ethylenamine (C₆-Produkt und C₄-Produkt) TETA und DETA (2. Fall) sowie als Nebenkomponenten weitere Ethylenamine enthält.

Die Nebenkomponenten können in beiden Fällen sowohl lineare als auch cyclische Ethylenamine oder sonstige Nebenprodukte sein. Als wichtigstes cyclisches Nebenprodukt wird im 1. Fall AEPip (C₆-(Neben)Produkt) gebildet. Das Verhältnis von TETA zu AEPip im Produkt liegt normalerweise zwischen 3:1 bis 12:1. Dieses Verhältnis kann beispielsweise durch die Wahl des Lösungsmittels, des Katalysators und/oder die Zugabe eines Additivs gesteuert werden. Im 1. Fall ist DETA ebenfalls ein (lineares) Nebenprodukt. Als weitere Nebenreaktionen gibt es Zersetzungsreaktionen, die aber insbesondere durch die Wahl des Lösungsmittels, der Zudosiergeschwindigkeit, der Eduktreinheit und/oder Katalysators gesteuert und minimiert werden können. Im 2. Fall tritt Pip als weiteres wichtiges cyclisches Nebenprodukt (C₄-(Neben)Produkt) auf, das hauptsächlich aus EDMN gebildet ist. Bezüglich der Bildung und Steuerung des Verhältnisses DETA zu Pip gelten die gleichen Aussagen wie bei TETA zu AEPip. Das erfindungsgemäße Verfahren wird im nachfolgenden Schema 1 anhand des 2. Falls verdeutlicht, bei dem EDDN und EDMN gemeinsam beispielhaft ausgehend von FACH hergestellt werden.

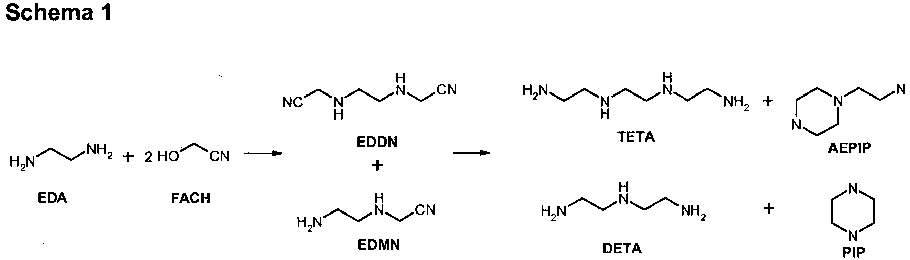

Im zweiten Fall wird der Begriff "Ethylenamingemisch" deswegen verwendet, weil das Reaktionsprodukt zwei lineare Ethylenamine als Hauptkomponenten (TETA und DETA) enthält, während im ersten Fall nur ein lineares Ethylenamin als Hauptprodukt (TETA) vorliegt. Die vorstehend bzw. nachfolgend aufgeführten Nebenprodukte werden folglich für die Begriffsdefinition in diesen beiden Fällen nicht berücksichtigt.

Im ersten Fall erhält man TETA mit einer Selektivität von vorzugsweise ≥ 70 Gew.-%, insbesondere ≥ 85 Gew.-%, bezogen auf die eingesetzte Menge an EDDN. Im zweiten Fall findet sich das Verhältnis der Edukte EDDN und EDMN prinzipiell nach der Hydrierung bei den entsprechenden Produkten TETA und DETA wieder.

Unter dem Begriff "weiteres Ethylenamin" soll im Rahmen der vorliegenden Erfindung jede von TETA (1. Fall) und von TETA und DETA (2. Fall) verschiedene kohlenwasserstoffhaltige Verbindung verstanden werden, die mindestens zwei Ethyleneinheiten und mindestens zwei funktionelle Gruppen enthält, wobei die funktionellen Gruppen ausgewählt sind aus einer primären, einer sekundären oder einer tertiären Aminogruppe. Als weiteres Ethylenamin sollen im Rahmen der vorliegenden Erfindung auch cyclische Verbindungen wie beispielsweise Piperazin (Pip) sowie dessen Derivate verstanden werden. Ebenfalls soll Ethylendiamin (EDA) als weiteres Ethylenamin aufgefasst werden. Weitere Ethylenamine sind insbesondere Diethylentriamin (DETA; nur 1. Fall), Piperazin (Pip), Aminoethylenpiperazin (AEPip) oder Tetraethylenpentamin (TEPA).

In einer Ausführungsform der vorliegenden Erfindung wird das bei der Hydrierung erhaltene DETA ganz oder teilweise nach Schritt a) zurückgeführt. Vorzugsweise wird diese Ausführungsform im Zusammenhang mit dem vorstehend beschriebenen zweiten Fall durchgeführt, in dem ein Ethylenamingemisch erhalten wird, enthaltend TETA und DETA jeweils als Hauptkomponenten. Zurückführung nach Schritt a) bedeutet somit, dass DETA zur Herstellung von Aminonitrilen, insbesondere von DETDN, verwendet wird, die wiederum anschließend hydriert werden.

Vorzugsweise wird DETA (ganz oder teilweise) zurückgeführt, so dass es gemäß Option a1) mit FACH umgesetzt wird. Dabei kann die Umsetzung von DETA mit FACH gleichzeitig mit der Umsetzung von EDA und FACH durchgeführt werden. Alternativ kann die Rückführung von DETA auch so durchgeführt werden, dass eine Teilmenge des eingesetzten FACH's mit DETA umgesetzt wird und eine andere Teilmenge mit EDA.

Durch die Umsetzung von DETA mit FACH wird hauptsächlich das Aminonitril Diethylentriamindiacetonitril (DETDN) hergestellt. Die Umsetzungsbedingungen von DETA mit FACH entsprechen weitgehend den vorstehend aufgeführten Umsetzungsbedingungen für EDA mit FACH. Vorzugsweise beträgt das molare Verhältnis von DETA zu FACH 1 : 1,5 bis 1 : 2 [mol/mol]. DETDN ist bis jetzt in der Literatur noch nicht beschrieben worden. Die Anmelderin der vorliegenden Anmeldung hat zeitgleich eine weitere Anmeldung eingereicht, die ein Verfahren zur Herstellung sowie Hydrierung von DETDN zum Gegenstand hat. Mit der Herstellung von DETDN kann als Nebenprodukt zusätzlich Diethylentriaminmonoacetonitril (DETMN) gebildet werden. Je nach Verhältnis des molaren Anteils von DETA zu FACH werden Aminonitrilgemische hergestellt enthaltend DETDN und DETMN. Bei einem nur geringen Überschuss von FACH zu DETA wird mehr DETMN neben DETDN gebildet.

Das Aminonitrilgemisch enthaltend DETDN sowie gegebenenfalls DETMN, hergestellt durch Umsetzung von DETA mit FACH, wird anschließend mit dem gemeinsam oder gegebenenfalls getrennt synthetisierten EDDN, das gegebenenfalls auch EDMN enthalten kann, vereinigt und hydriert (sofern DETA und EDA getrennt voneinander mit FACH umgesetzt worden sind).

Bei der anschließenden Hydrierung dieser Ausführungsform des erfindungsgemäßen Verfahrens, bei der neben EDDN und EDMN weiterhin DETDN sowie gegebenenfalls DETMN gemeinsam hydriert werden, wird ein Ethylenamingemisch erhalten, das als Hauptkomponenten TETA und DETA sowie zusätzlich TEPA enthält. An dieser Stelle sei darauf hingewiesen, dass auch aus DETMN bei der Hydrierung TETA gebildet wird. Die Anwesenheit von EDMN bei der Hydrierung ist erforderlich, um das gebildete DETA ganz oder teilweise wieder rückführen zu können. Das nachfolgende Schema 2 liefert einen Überblick über diese Ausführungsform des erfindungsgemäßen Verfahrens hinsichtlich des Teilschrittes der Umsetzung von DETA mit FACH. Die parallel stattfindende Herstellung sowie Hydrierung von EDDN und EDMN ist in diesem Schema nicht dargestellt.

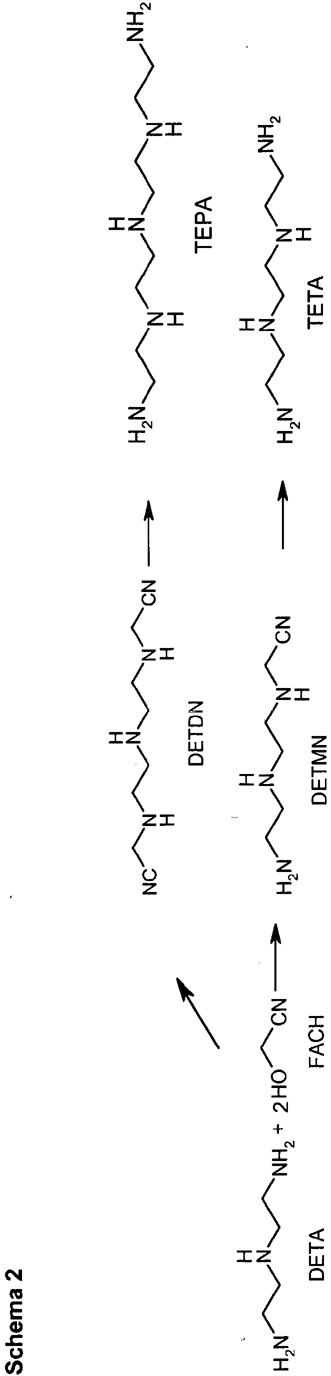

In einer weiteren Ausführungsform der vorliegenden Erfindung wird das bei der Hydrierung als Nebenprodukt erhaltene AEPip ganz oder teilweise nach Schritt a) zurückgeführt. Vorzugsweise wird diese Ausführungsform im Zusammenhang mit dem vorstehend beschriebenen zweiten Fall durchgeführt, in dem ein Ethylenamingemisch erhalten wird, enthaltend TETA und DETA jeweils als Hauptkomponenten. Die Zurückführung kann dabei zusammen oder getrennt von der vorstehend beschriebenen Zurückführung von DETA erfolgen. Vorzugsweise erfolgt die Zurückführung von AEPip zusammen mit DETA. Zurückführung nach Schritt a) bedeutet somit, dass AEPip zur Herstellung von cyclischen Aminonitrilen verwendet wird, die wiederum anschließend hydriert werden unter Erhalt von cyclischen Ethylenaminen (Piperazinderivate höherer Ethylenamine).

Vorzugsweise wird AEPip (ganz oder teilweise) zurückgeführt, so dass es gemäß Option a1) mit FACH umgesetzt wird. Dabei kann die Umsetzung von AEPip mit FACH gleichzeitig mit der Umsetzung von EDA und/oder DETA mit FACH durchgeführt werden. Alternativ kann die Rückführung von AEPip auch so durchgeführt werden, dass eine Teilmenge des eingesetzten FACH's mit AEPip umgesetzt wird und weitere Teilmengen mit EDA und/oder DETA.

Durch die Umsetzung von AEPip mit FACH werden hauptsächlich die cyclischen Aminonitrile Piperazinylethylaminoacetonitril (PEAN), Aminoethylpiperazinylacetonitril (AEPAN) und/oder Cyanomethylpiperazinylethylaminoacetonitril (CMPEAN) hergestellt. Die Umsetzungsbedingungen von AEPip mit FACH entsprechen prinzipiell den vorstehend aufgeführten Umsetzungsbedingungen für DETA mit FACH. Vorzugsweise beträgt das molare Verhältnis von AEPip zu FACH 1 : 1,5 bis 1 : 2 [mol/mol].

Die bei der Umsetzung von AEPip mit FACH entstehenden cyclischen Aminonitrile PEAN, AEPAN und CMPEAN sind neue Verbindungen, die in der Literatur noch nicht beschrieben sind. Demzufolge sind diese 3 cyclischen Aminonitrile als solche oder eines Gemisches davon sowie Verfahren zu deren Herstellung weitere Gegenstände der vorliegenden Erfindung.

Das Aminonitrilgemisch enthaltend PEAN, AEPAN und/oder CMPEAN, hergestellt durch Umsetzung von AEPip mit FACH, wird anschließend mit dem getrennt synthetisierten EDDN, das gegebenenfalls auch EDMN enthalten kann und/oder dem getrennt synthetischen DETDN, das gegebenenfalls auch DETMN enthalten kann, vereinigt und hydriert (sofern AEPip sowie DETA und/oder EDA getrennt voneinander mit FACH umgesetzt worden sind).

Bei der anschließenden Hydrierung dieser Ausführungsform des erfindungsgemäßen Verfahrens, bei der neben EDDN und EDMN weiterhin DETDN, PEAN, AEPAN und/oder CMPEAN sowie gegebenenfalls DETMN gemeinsam hydriert werden, wird ein Ethylenamingemisch erhalten, das als Hauptkomponenten TETA und DETA sowie zusätzlich TEPA, Diaminoethylpiperazin (DAEPip), Piperazinethylethylendiamin (PEEDA) und/oder Aminoethylpiperazinethylethylendiamin (AEPEEDA) enthält. Die Anwesenheit von EDMN bei der Hydrierung ist nur dann erforderlich, um über das gebildete DETA nach der Rückführung DETDN und daraus TEPA als weitere Hauptkomponente herzustellen. Das nachfolgende Schema 3 liefert einen Überblick über diese Ausführungsform des erfindungsgemäßen Verfahrens hinsichtlich des Teilschrittes der Umsetzung von AEPip mit FACH. Die parallel stattfindende Herstellung sowie Hydrierung von EDDN und EDMN bzw. DETDN und DETMN ist in diesem Schema nicht dargestellt. Die cyclischen Ethylenamine AEPEEDA, DAEPip und PEEDA sind bekannte Nebenprodukte bei der großtechnischen Darstellung von TETA oder TEPA nach dem EDC-Prozess. Bevorzugte cyclische Ethylenamine sind bei dieser Ausführungsform DAEPip und PEEDA.

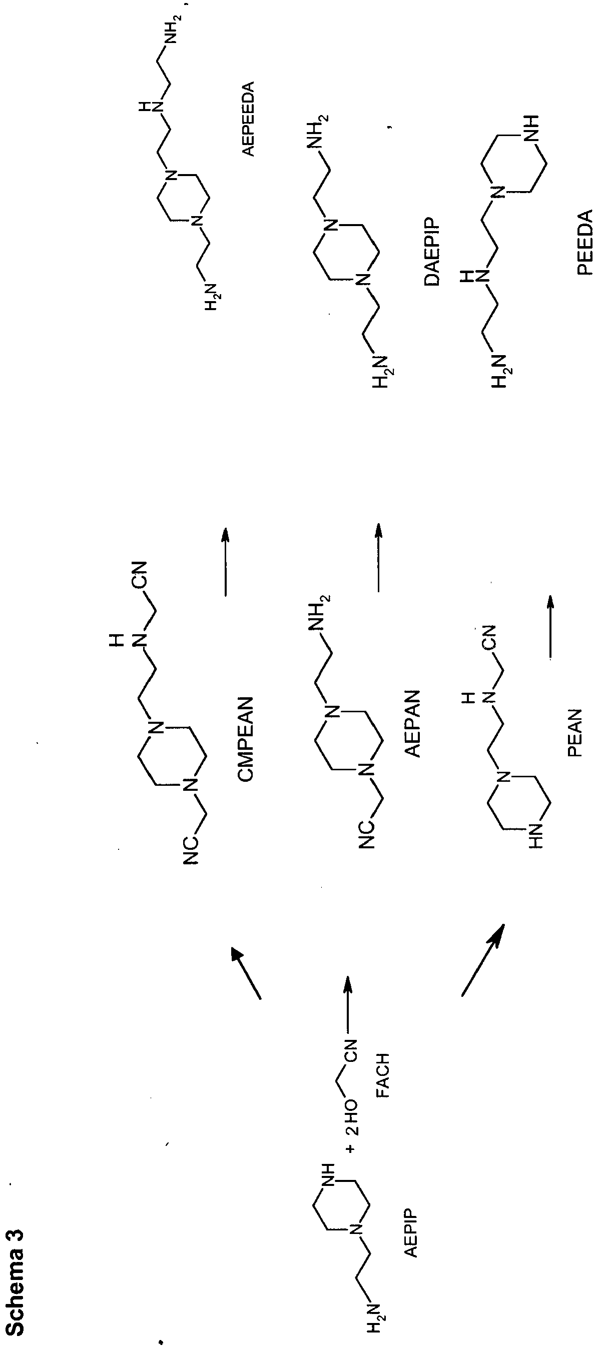

Im Anschluss an die Hydrierung kann das erhaltene Produkt (TETA oder Ethylenamingemisch) gegebenenfalls weiter aufgereinigt werden, beispielsweise indem das Lösungsmittel und/oder der Katalysator nach dem Fachmann bekannten Methoden abgetrennt werden. Insbesondere können die Hauptprodukte (TETA und gegebenenfalls DETA, TEPA oder gegebenenfalls die cyclischen Ethylenamine DAEPip, PEEDA und/oder AEPEEDA) gemeinsam oder einzeln nach dem Fachmann bekannten Methoden aus dem Reaktionsprodukt isoliert werden. Sofern die jeweiligen Hauptprodukte gemeinsam isoliert werden, beispielsweise durch eine Destillation, können sie anschließend in die jeweiligen Einzelprodukte isoliert werden. Letztendlich erhält man somit reines TETA, reines DETA, reines TEPA sowie gegebenenfalls reines DAEPip, reines PEEDA und/oder reines AEPEEDA). Sonstige Verunreinigungen, Nebenprodukte oder weitere Ethylenamine wie TEPA oder Pip können ebenfalls mit dem Fachmann bekannten Methoden aus dem jeweiligen Produkt abgetrennt werden, sofern sie vorhanden sind.

Gegebenenfalls können auch Gemische von 2 oder mehr der vorgenannten Ethylenamine isoliert, beispielsweise als Gemische mit TETA und/oder TEPA.

In einer bevorzugten Ausführungsform wird das erfindungsgemäße Verfahren unter Verwendung von Tetrahydrofuran oder Methanol als Lösungsmittel durchgeführt. Die Temperatur bei der Hydrierung beträgt vorzugsweise 80 bis 140°C, der Druck vorzugsweise 40 bis 160 bar. Vorzugsweise wird die Hydrierung in Anwesenheit von EDA und/oder gegebenenfalls von Ammoniak durchgeführt.

Mit dem erfindungsgemäßen Verfahren wird neben hohen Gesamtausbeuten an Ethylenaminen ein hoher Anteil an linearem TETA bzw. weiteren linearen Ethylenaminen erzielt.

Die nachfolgenden Beispiele verdeutlichen das erfindungsgemäße Verfahren. Die Anteile sind in Gew.-% angegeben, sofern nicht anders aufgeführt. Ein mitgeführter interner Standard, Diethylenglykoldimethylether (DEGDME), erlaubt eine Quantifizierung des Produktes durch Bestimmung der eventuell gebildeten flüchtigen Zersetzungsbestandteile. Die Quantifizierung erfolgt mittels Gaschromatographie (GC), wobei den jeweils entnommenen Proben zur Homogenisierung Methanol zugegeben wird.

### Beispiele

### Allgemeine Vorschrift zur Synthese von Formaldehydcyanhydrin (FACH)

### Variante a)

In einem 6l-Reaktionsgefäß mit Propellerrührer werden 6000 g (60 mol) Formaldehyd (30 %) vorgelegt und mit Natronlauge (1 mol/l) ein pH-Wert von 5.5 eingestellt. Innerhalb 2.5 Stunden werden 1661 g (61.2 mol) Blausäure über ein beheiztes U-Rohr unterhalb des Rührers gasförmig eindosiert, wobei die Reaktionstemperatur bei 30 °C und der pH-Wert bei 5.5 gehalten wird. Nach 30 Minuten Nachrührzeit wird der pH-Wert mit Schwefelsäure (50 %ig) auf 2.5 gestellt. Über Liebig-Titration wird der entsprechende Gehalt ermittelt.

### Variante b)

In einem 6l-Reaktionsgefäß mit Propellerrührer werden 7000 g (70 mol) Formaldehyd (30 %) vorgelegt und mit Natronlauge (1 mol/l) ein pH-Wert von 5.5 eingestellt. Innerhalb 3 Stunden werden 1938 g (71,4 mol) Blausäure über ein auf 50 °C beheiztes U-Rohr unterhalb des Rührers gasförmig eindosiert, wobei die Reaktionstemperatur bei 30 °C und der pH-Wert bei 5.5 gehalten wird. Nach 10 Minuten Nachrührzeit wird der pH-Wert mit Schwefelsäure (50 %ig) auf 2.5 gestellt. Um Leichtsieder, insbesondere Blausäure abzutrennen, wird der Reaktionsaustrag einer Sambay-Destillation (wie in "Chemie Ingenieur Technik, Vol. 27, S. 257-261 beschrieben) (1 mbar, 30 °C) unterzogen. Über Liebig-Titration wird der entsprechende Gehalt ermittelt und gegebenenfalls durch Zugabe von Wasser ein Gehalt von 43-44 % bzw. 67 % FACH eingestellt.

### Beispiel 1:

### Formaldehydcyanhydrin

FACH wird nach der allgemeinen Vorschrift nach Variante a) hergestellt.

### Ethylendiamindiacetonitril

In einem 21-Reaktionsgefäß werden 536.5 g (4 mol) FACH (42.5 %) vorgelegt und unter Eiskühlung bei einer Temperatur von maximal 35 °C innerhalb von 2 Stunden 132 g (2.2 mol) Ethylendiamin zugetropft. Der Reaktionsansatz verfärbt sich von leicht gelb über orange nach braun. Nach kurzer Nachrührzeit wird die freie Blausäure durch Strippen mit Stickstoff entfernt (Volhard-Titration). Es wird laut Liebig-Titration ein Umsatz an FACH von 97.2 % erhalten.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 4,2g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 28% AEPip sowie 30% TETA. Des Weiteren werden 4 Gew.-% an C4-Produkten (Pip + DETA) gefunden.
b) Die gleiche Ware wird ebenfalls im Semi-Batch-Verfahren hydriert. Hierbei werden in einem 270mL-Autoklaven 3,25g eines Cr-dotierten Raney-Cobalt Katalysators sowie 15ml THF und 5,4g EDA vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 4,2g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 12% AEPip sowie 43% TETA. Des Weiteren wurden 4 Gew.-% an C4-Produkten (Pip + DETA) gefunden.

Im Vergleich zu Beispiel 1a zeigt sich ein positiver Einfluss von EDA auf die TETA-Bildung.

### Beispiel 2:

### Formaldehydcyanhydrin

FACH wird nach der allgemeinen Vorschrift nach Variante b) hergestellt.

### Ethylendiamindiacetonitril

In einem 21-Reaktionsgefäß werden 132 g (2.2 mol) EDA vorgelegt und unter Eiskühlung bei einer Temperatur von maximal 30 °C innerhalb von 2 Stunden 511.2 g (4 mol) FACH (44.6 %) zugetropft. Nach 4.5 Stunden Nachrührzeit wird die leicht gelbe Lösung abgefüllt. Der Umsatz FACH beträgt laut Liebig-Titration 99.2 %. Der Reaktionsansatz enthält 0.11 % freie Blausäure (bestimmt durch Volhard-Titration). Durch Titration wird eine EDDN-Ausbeute von 91,7 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 95,7 % und somit eine Ausbeute an EDMN von 4 %.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 4,2g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 27% AEPip sowie 47% TETA. Des Weiteren werden 8 Gew.-% an C4-Produkten nachgewiesen.

Es zeigt sich, dass durch die Leichtsieder-Abtrennung nach der FACH-Synthese eine deutlich bessere Ausbeute an Ethylenaminen erzielt werden kann. Durch den Überschuss an EDA in der EDDN-Synthese wird EDMN gebildet, welches zu den C4-Produkten DETA und Pip hydriert wird.
b) Die gleiche Ware wird ebenfalls im Semi-Batch hydriert. Hierbei werden in einem 270mL-Autoklaven 3,25g eines Cr-dotierten Raney-Cobalt Katalysators sowie 15ml THF und 13,5g EDA vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 4,2g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 8% AEPip sowie 82% TETA. Des Weiteren werden 16 Gew.-% an C4-Produkten nachgewiesen.

Durch die EDA-Zugabe in Variante 2b) wird mehr lineares TETA gebildet. Ebenfalls zeigt sich ein Anstieg an C4-Produkten, der auf EDA-Kondensation beruht. Bei der Gew.-%-Angabe der C₄-Produkte ist die Gewichtszunahme durch EDA-Kondensation mit berücksichtigt.

### Beispiel 3:

### Formaldehydcyanhydrin

FACH wird nach der allgemeinen Vorschrift nach Variante b) hergestellt.

### Ethylendiamindiacetonitril

In einem 21-Reaktionsgefäß werden 132 g (2.2 mol) EDA vorgelegt und unter Eiskühlung bei einer Temperatur von maximal 30 °C innerhalb von etwa 2 Stunden 340,8 g (4 mol) FACH (67 %) zugetropft. Nach 3 Stunden Nachrührzeit wird die gelbe Lösung abgefüllt. Der Umsatz FACH beträgt laut Liebig-Titration 99.5 %. Der Reaktionsansatz enthält 0.08 % freie Blausäure (bestimmt durch Volhard-Titration). Durch Titration wird eine EDDN-Ausbeute von 82,9 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 90,5 % und somit eine Ausbeute an EDMN von 8 %.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 10g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 10% AEPip sowie 69% TETA. Zusätzlich werden 13% an C4-Produkten (Pip und DETA) erhalten.

Für die Vergleichbarkeit wird gegenüber Beispiel 2a mehr Wasser zugegeben. Durch den Überschuss an EDA in der EDDN-Synthese wird EDMN gebildet, welches zu den C4-Produkten DETA und Pip hydriert wird.
b) Die erhaltene Ware wird im Semi-Batch-Verfahren hydriert. Hierbei werden in einem 270mL-Autoklaven 3,25g eines Cr-dotierten Raney-Cobalt Katalysators, 15ml THF und 13,5g EDA vorgelegt. Der Autoklav wird auf 120°C hochgeheizt und Wasserstoff bis zu einem Gesamtdruck von 100bar aufgepresst. Innerhalb von 120 min wird eine Mischung aus 13,8g der Roh-EDDN-Lösung, 13,8g eines internen Standards sowie 10g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 5% AEPip sowie 76% TETA. Des Weiteren werden 16% an C4-Produkten erhalten.

Durch die EDA-Zugabe wird mehr lineares TETA gebildet. Ebenfalls zeigt sich ein Anstieg an C4-Produkten, was auf EDA-Kondensation beruht.
c) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 9% AEPip sowie 76% TETA. Zusätzlich werden 12% an C4-Produkten (Pip und DETA) erhalten.

Im Vergleich zu Beispiel 3a wird auf eine zusätzliche Zugabe an Wasser verzichtet, was sich positiv auf TETA auswirkt.

### Beispiel 4:

### Formaldehydcyanhydrin

FACH wird nach der allgemeinen Vorschrift nach Variante b) hergestellt.

### Ethylendiamindiacetonitril

In einem 21-Reaktionsgefäß werden 132 g (2.2 mol) EDA vorgelegt und unter Eiskühlung bei einer Temperatur von maximal 50 °C innerhalb von 35 Minuten 340,8 g (4 mol) FACH (67 %) zugetropft. Nach 1 Stunde Nachrührzeit wird die fast klare Lösung abgefüllt. Der Umsatz FACH beträgt laut Liebig-Titration 99.2 %. Der Reaktionsansatz enthält 0.07 % freie Blausäure (bestimmt durch Volhard-Titration). Durch Titration wird eine EDDN-Ausbeute von 87,7 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 93 % und somit eine Ausbeute an EDMN von 5 %.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 10% AEPip sowie 76% TETA. Zusätzlich werden 11 % an C4-Produkten (Pip und DETA) erhalten.

Versuch 4a bestätigt die Ergebnisse von 3c. Auch hier liegt durch den Überschuss an EDA in der EDDN-Synthese die Ausbeute an C4-Produkten (DETA und Pip) bei ca. 11%.
b) Die erhaltene Ware wird im Semi-Batch-Verfahren hydriert. Hierbei werden in einem 270mL-Autoklaven 3,25g eines Cr-dotierten Raney-Cobalt Katalysators, 15ml THF und 13,5g EDA vorgelegt. Der Autoklav wird auf 120°C hochgeheizt und Wasserstoff bis zu einem Gesamtdruck von 100bar aufgepresst. Innerhalb von 120 min wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 4% AEPip sowie 80% TETA. Des Weiteren werden 15% an C4-Produkten erhalten.

In Beispiel 4b wird bestätigt, dass durch die Hydrierung in Gegenwart von EDA und einer geringeren Wassermenge, die AEPip-Bildung deutlich unterdrückt werden kann. Der Gehalt an 15 Gew.-% an C4-Produkten ist üblich, bei dem vorliegenden EDA-Überschuss in der EDDN-Synthese sowie EDA in der Hydrierung.

### Beispiel 5:

### Formaldehydcyanhydrin

FACH wird nach der allgemeinen Vorschrift nach Variante b) hergestellt.

### Ethylendiamindiacetonitril

In einem 21-Reaktionsgefäß werden 180 g (3 mol) EDA vorgelegt und unter Eiskühlung bei einer Temperatur von maximal 50 °C innerhalb von etwa 1 Stunde 511.2 g (6 mol) FACH (67 %) zugetropft. Nach 1,5 Stunden Nachrührzeit wird die hellgelbe Lösung abgefüllt. Der Umsatz FACH beträgt laut Liebig-Titration 99,2 %. Der Reaktionsansatz enthält 0.02 % freie Blausäure (bestimmt durch Volhard-Titration). Durch Titration wird eine EDDN-Ausbeute von 92,6 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 94,5 % und somit eine Ausbeute an EDMN von 2 %.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 10% AEPip sowie 77% TETA. Zusätzlich werden 3% an C4-Produkten (Pip und DETA) erhalten.

Es zeigt sich, dass durch den Einsatz von halb-molaren Mengen an EDA bei der EDDN-Herstellung der Gehalt an C4-Produkten nach der Hydrierung lediglich 3% beträgt.
b) Die erhaltene Ware wird im Semi-Batch-Verfahren hydriert. Hierbei werden in einem 270mL-Autoklaven 3,25g eines Cr-dotierten Raney-Cobalt Katalysators, 15ml THF und 13,5g EDA vorgelegt. Der Autoklav wird auf 120°C hochgeheizt und Wasserstoff bis zu einem Gesamtdruck von 100bar aufgepresst. Innerhalb von 120 min wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 6% AEPip sowie 82% TETA. Des Weiteren werden 7% an C4-Produkten erhalten.

Auch hier liegt der Gehalt an C4-Produkten deutlich unter demjenigen von Beispiel 4b.

Die vorstehenden Beispiele zeigen, dass die Qualität des eingesetzten FACHs Einfluss auf die Reaktionsdauer und die Farbe des Produktes bei der EDDN-Herstellung hat. Bei der anschließenden Hydrierung wird zudem eine höhere Selektivität erzielt, sofern das FACH destillativ aufgereinigt wird. Die Zugabe eines Additivs wirkt sich weiterhin positiv auf die Selektivität hinsichtlich linearer Ethylenamine aus. Die Wassermenge zeigt ebenfalls Einfluss auf die Bildung an linearem TETA.

### Beispiel 6:

### Formaldehydcyanhydrin

FACH wird nach der allgemeinen Vorschrift nach Variante b) hergestellt.

### Ethylendiamindiacetonitril

In einem 21-Reaktionsgefäß werden 132 g (2.2 mol) EDA vorgelegt und unter Eiskühlung bei einer Temperatur von maximal 50 °C innerhalb von 35 Minuten 340,8 g (4 mol) FACH (67 %) zugetropft. Nach 1 Stunde Nachrührzeit wird die fast klare Lösung abgefüllt. Der Umsatz FACH beträgt laut Liebig-Titration 99.2 %. Der Reaktionsansatz enthält 0.07 % freie Blausäure (bestimmt durch Volhard-Titration). Durch Titration wird eine EDDN-Ausbeute von 87,7 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 93 % und somit eine Ausbeute an EDMN von 5 %.

### Triethylentetraamin

Die anschließende Hydrierung der oben erhaltenen Lösung wird kontinuierlich in einem 270mL Autoklav mit Stromstörern und Scheibenrührer durchgeführt. Hierbei werden 22g Cr-dotierter Raney-Cobalt vorgelegt und kontinuierlich 20NL Wasserstoff zugefahren. Pro Stunde werden 4,5g der EDDN-Lösung zusammen mit 2g eines internen Standards, 4,9g EDA sowie 30g THF zugefahren. Die Hydrierung wird bei 120°C und 100bar durchgeführt. Über einem Zeitraum von 26h können im Durchschnitt 2,6 Gew.-% Pip, 19,5 Gew.-% DETA an C4-Produkten sowie 5,6 Gew.-% AEPip und 79,9 Gew.-% TETA an C6-Produkten isoliert werden. Bezüglich EDDN entspricht dies einer Ausbeute von 96% an C6-Produkten.

### Beispiel 7:

### Formaldehydcyanhydrin

FACH wird nach der allgemeinen Vorschrift nach Variante b) hergestellt.

### Ethylendiamindiacetonitril

In einem 21-Reaktionsgefäß werden 120 g (2 mol) EDA vorgelegt und unter Eiskühlung bei einer Temperatur von maximal 70 °C innerhalb von 30 Minuten 340,8 g (4 mol) FACH (67 %) zugetropft. Nach 1 Stunde Nachrührzeit wird die klare gelb-orange Lösung abgefüllt. Der Umsatz FACH beträgt laut Liebig-Titration 99,3 %. Der Reaktionsansatz enthält 0,12 % freie Blausäure (bestimmt durch Volhard-Titration). Durch Titration wird eine EDDN-Ausbeute von 91,6 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 94,3 % und somit eine Ausbeute an EDMN von 3 %.

### Triethylentetraamin

Die anschließende Hydrierung der oben erhaltenen Lösung wird kontinuierlich in einem 270mL Autoklav mit Stromstörern und Scheibenrührer durchgeführt. Hierbei werden 22g Cr-dotierter Raney-Cobalt vorgelegt und kontinuierlich 20NL Wasserstoff zugefahren. Pro Stunde werden 4,5g der EDDN-Lösung zusammen mit 2g eines internen Standards, 4,9g EDA sowie 30g THF zugefahren. Die Hydrierung wird bei 120°C und 100 bar durchgeführt. Über einem Zeitraum von 26h können im Durchschnitt 2,4 Gew.-% Pip, 13,2 Gew.-% DETA an C4-Produkten sowie 4,8 Gew.-% AEPip und 84,1 Gew.-% TETA an C6-Produkten isoliert werden. Bezüglich EDDN entspricht dies einer Ausbeute von 98% an C6-Produkten.

### Beispiel 8: Belastungseinfluss in der Hydrierung

In einer eigenen Versuchsreihe wird der Einfluss der Belastung lediglich auf das Verhältnis TETA/AEPip betrachtet.

### Formaldehydcyanhydrin

FACH wird nach der allgemeinen Vorschrift nach Variante a) hergestellt.

### Ethylendiamindiacetonitril

In einem 21-Reaktionsgefäß werden 132 g (2,2 mol) EDA vorgelegt und unter Eiskühlung bei einer Temperatur von maximal 35 °C innerhalb von 1,5 Stunden 506,6 g (4 mol) FACH (45 %ig) zugetropft. Nach 1 Stunde Nachrührzeit werden 14,3 g (0,1 mol) FACH (45 %ig) nachdosiert und auf 40 °C erwärmt. Es wird laut Liebig-Titration ein Umsatz an FACH von etwa 100 % erhalten.

### (EDDN-Hydrierung verschiedene Belastungen):

In einem 270mL-Autoklaven wird 3,25g (trocken) eines Cr-dotierten Raney-Cobalt Katalysators sowie 15ml THF vorgelegt. Der Autoklav wird auf 120°C hochgeheizt und Wasserstoff bis zu einem Gesamtdruck von 200bar aufgepresst. Innerhalb von einem definierten Zeitraum wird 13,8g der oben erhaltenen wässrigen EDDN-Lösung, 13,8g eines internen Standards sowie 4,2 g Wasser in 106 g THF zudosiert. Die Reaktionsmischung wird weitere 60 Minuten unter Reaktionsbedingungen gerührt. Zu unterschiedlichen Zeiträumen werden Proben genommen, die mittels Methanol homogenisiert werden. Nach vollständiger Zugabe kann kein EDDN nachgewiesen werden.

Das Verhältnis an TETA/AEPip wird bestimmt gemäß:
a) 60min Zugabe: TETA/AEPip: 2,2
b) 180min Zugabe: TETA/AEPip: 3,3
c) 180min Zugabe: TETA/AEPip: 4,5

Bei 80°C Hydriertemperatur und 60min Zudosierung kann lediglich ein TETA/AE-Pip-Verhältnis von 1,3 erzielt werden.

### Beispiel 9 (Ammoniak als Additiv):

Für die Hydrierung in Gegenwart von Ammoniak wird die in Beispiel 7 erhaltene EDDN-Lösung eingesetzt.
a) In einem 270mL-Autoklaven werden 3,25g (trocken) eines Cr-dotierten Raney-Cobalt Katalysators, 15ml THF und 5,2g EDA vorgelegt. Der Autoklav wird auf 120°C hochgeheizt und Wasserstoff bis zu einem Gesamtdruck von 200bar aufgepresst. Innerhalb von 60 Minuten werden 13,8g der oben erhaltenen wässrigen EDDN-Lösung (43 Gew.-%), 13,8g eines internen Standards sowie 4,2g Wasser in 106g THF zudosiert. Die Reaktionsmischung wird weitere 60 Minuten unter Reaktionsbedingungen gerührt. Zu unterschiedlichen Zeiträumen werden Proben genommen, die mittels Methanol homogenisiert werden. Nach vollständiger Zugabe kann kein EDDN nachgewiesen werden. Nach 60minütiger Nachhydrierzeit liegt das Verhältnis an TETA und AEPip bei 4,1.
   In einem weiteren Versuch wird zusätzlich zum EDA 12g Ammoniak vorgelegt. Hierdurch konnte das Verhältnis auf 9,0 erhöht werden.
b) In einem 270mL-Autoklaven werden 3,25g (trocken) eines Cr-dotierten Raney-Cobalt Katalysators, 15ml THF und 12g Ammoniak vorgelegt. Der Autoklav wird auf 120°C hochgeheizt und Wasserstoff bis zu einem Gesamtdruck von 200bar aufgepresst. Innerhalb von 60 Minuten werden 13,8g der oben erhaltenen wässrigen EDDN-Lösung (43 Gew.-%), 13,8g eines internen Standards sowie 4,2g Wasser in 106g THF zudosiert. Die Reaktionsmischung wird weitere 60 Minuten unter Reaktionsbedingungen gerührt. Zu unterschiedlichen Zeiträumen werden Proben genommen, die mittels Methanol homogenisiert werden. Nach vollständiger Zugabe kann kein EDDN nachgewiesen werden. Nach 60minütiger Nachhydrierzeit liegt das Verhältnis an TETA und AEPip bei 5,7

## Patentansprüche

1. Aminonitril ausgewählt aus Piperazinylethylaminoacetonitril (PEAN), Aminoethylpiperazinylacetonitril (AEPAN) oder Cyanomethylpiperazinylethylaminoacetonitril (CMPEAN).

2. Verfahren zur Herstellung eines Aminonitrils gemäß Anspruch 1, **dadurch gekennzeichnet, dass** AEPip mit FACH umgesetzt wird.

## Claims

1. An amino nitrile selected from among piperazinylethylaminoacetonitrile (PEAN), aminoethylpiperazinylacetonitrile (AEPAN) and cyanomethylpiperazinylethylaminoacetonitrile (CMPEAN).

2. A process for preparing an amino nitrile according to claim 1, which comprises reacting AEPip with FACH.

## Revendications

1. Aminonitrile choisi parmi le pipérazinyléthylaminoacétonitrile (PEAN), l'aminoéthylpipérazinylacétonitrile (AEPAN) ou le cyanométhylpipérazinyléthylaminoacétonitrile (CMPEAN).

2. Procédé pour la préparation d'un aminonitrile selon la revendication 1, **caractérisé en ce qu'**on transforme de l'AEPip avec de la FACH.
